# EUROPEAN PATENT APPLICATION

(11) **EP 1 849 523 A1**
(43) Date of publication of application: **31.10.2007**
(21) Application number: 06712367.9
(22) Date of filing: 26.01.2006
(51) Int. Cl.: B03C 3/40, A61L 9/00, A61L 9/22, B03C 3/155, B03C 3/41, B03C 3/70

(54) **AIR CLEANER**

(30) Priority: 14.02.2005 JP 2005036689
(71) Applicant: DAIKIN INDUSTRIES, LTD., Osaka-shi, Osaka 530-8323 (JP)
(72) Inventor: MOTEGI, Kanji, Daikin Industries, Ltd., Sakai-shi, Osaka 5918511 (JP); AKIYAMA, Ryuji, Daikin Industries, Ltd., Sakai-shi, Osaka 5918511 (JP); TANAKA, Toshio, Daikin Industries, Ltd., Sakai-shi, Osaka 5918511 (JP); KAGAWA, Kenkichi, Daikin Industries, Ltd., Sakai-shi, Osaka 5918511 (JP)
(74) Representative: Goddar, Heinz J.
(86) International application number: PCT/JP2006/301185
(87) International publication number: WO 2006/085439

(57) **Abstract**

Disclosed is an air purification device including a discharge part for discharging electricity in air. Low-profiling of the air purification device is accomplished while maintaining the discharge capability of the discharge part. In an ionization part **(30)** for electrically charging airborne dust particles, an insulating medium **(60)** is disposed between an ionization wire **(31)** serving as a discharge base end and an earth electrode **(33).** As a result of such arrangement, electrical discharge from the ionization wire **(31)** to the earth electrode **(33)** is obstructed while on the other hand corona discharge from the ionization wire **(31)** to a first electrode plate **(32)** is maintained, whereby the distance between the ionization wire **(31)** and the earth electrode **(33)** can be made narrow.

## Description

### TECHNICAL FIELD

The present invention relates to an air purification device including a discharge part for discharging electricity in air in which a stream of air to be treated flows.

### BACKGROUND ART

An air purification device for purifying air in an indoor space (living place, commercial store et cetera) has been known in the art.

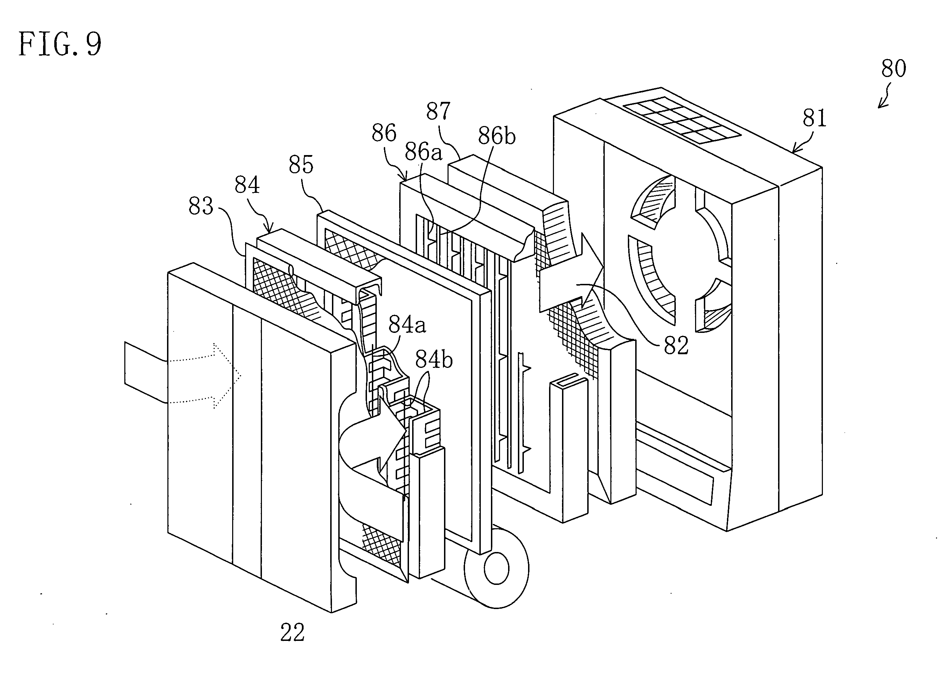

For example, a conventional air purification device **(80)** is shown in Figure **9,** in which air purification device an air passageway **(82)** through which a stream of air to be treated flows is formed within a casing **(81).** Disposed sequentially in the upstream to downstream direction of the flow of air in the air passageway **(82)** are a prefilter **(83),** an ionization part **(84),** an electrostatic filter **(85),** a streamer discharge part **(86),** and a catalytic filter **(87).**

The prefilter **(83)** traps dust particles of relatively large size present in the air introduced into the casing **(81).** The ionization part **(84)** is made up of a discharge electrode **(84a)** for electrical dust collection and a counter electrode **(84b).** The discharge electrode **(84a)** is made up of a plurality of linear electrodes and extends across a frame body from the upper to the lower side thereof. An electrode member having a cross section of squared U-shape is disposed around the discharge electrode **(84a),** in other words its upstream side is opened. Right and left side walls of the electrode member constitute the counter electrode **(84b).** In the ionization part **(84),** corona discharge is generated between the electrodes **(84a, 84b),** whereby dust particles present in the air to be treated are electrically charged. The dust particles thus electrically charged are electrically trapped by the electrostatic filter **(85).**

The streamer discharge part **(86)** is made up of a discharge electrode **(86a)** for streamer discharge and a counter electrode **(86b).** The discharge electrode **(86a)** is made up of a plurality of needle-like or projection-like electrodes formed on a plate-like member supported at the upper and lower ends of a frame body. On the other hand, the counter electrode **(86b)** is made up of a plurality of plate-like electrodes disposed on the lateral side of a supporting member so that it faces the discharge electrode **(86a).** In the streamer discharge part **(86),** streamer discharge is generated between the electrodes **(86a, 86b),** whereby so-called active species (radicals, fast electrons, excited molecules) are produced. Further activation of these generated active species is effected by the catalytic filter **(87),** thereby accomplishing decomposition of harmful components and odorous components present in the air to be treated (see JP-A-2004-329639).

### DISCLOSURE OF THE INVENTION

### PROBLEMS WHICH THE INVENTION SEEKS TO OVERCOME

In recent years, air purification devices of the above-described type for use in consumer application (living place, commercial store et cetera) have been in widespread use. Incidentally, it is preferable that such a consumer air purification device be designed to be as compact as possible. Especially, low-profiling of the air purification device is an effective improvement for efficient use of the residential space. On the other hand, if trying to make an air purification device having a discharge part of the above-described type (e.g., an ionization part or a streamer discharge part) low profiled, the distance between the discharge electrode and the other component may be narrowed in some cases.

For example, if trying to low-profile the air purification device **(80)** disclosed in the aforesaid patent document, this gives rise to the possibility that the distance between the discharge electrode **(84a)** of the ionization part **(84)** and the electrostatic filter **(85)** may be narrowed. In this case, in addition to the electrical discharge from the discharge electrode **(84a)** to the counter electrode **(84b),** electrical discharge from the discharge electrode **(84a)** to the electrostatic filter **(85)** is possible. This gives rise to the possibility of generating a spark to the electrostatic filter **(85)** from the discharge electrode **(84a).** In addition, a desired corona discharge is not generated in the ionization part **(84),** thereby giving rise to the possibility that the capability of collecting dust particles present in the air to be treated may fall.

In addition, if trying to low-profile the air purification device **(80),** the distance between the discharge electrode **(86a)** of the streamer discharge part **(86)** and the other component may be narrowed. In this case, a desired streamer discharge is not generated because of electrical discharge from the discharge electrode **(86a)** to the other component, thereby giving rise to the possibility that the efficiency of decomposing harmful/odorous components present in the air to be treated may fall.

With the above problems with the conventional technology in mind, the present invention was devised. Accordingly, a general object of the present invention is to achieve low-profiling of an air purification device having a discharge part for discharging electricity in air while maintaining the discharge capability of the discharge part.

### MEANS FOR OVERCOMING THE PROBLEMS

The present invention provides, as a first aspect, an air purification device which comprises an air passageway **(15)** through which air to be treated flows and a discharge part **(30, 40)** which discharges electricity in the air flowing through the air passageway **(15).** The air purification device of the first aspect is characterized in that an insulating medium **(60)** is disposed between the discharge part **(30, 40)** and a component **(16, 17, 33)** adjacent to the discharge part **(30, 40).** The aforesaid "discharge part" is provided to generate interelectrode electrical discharge for purifying air to be treated, the interelectrode electrical discharge including corona discharge, streamer discharge, glow discharge et cetera. In addition, by "component" is meant a component other than the discharge part **(30, 40)** disposed in the air passageway **(15),** especially one that is disposed adjacent to the discharge part **(30, 40).**

In the first aspect of the present invention, the insulating medium **(60)** is so disposed as to provide isolation between the discharge part **(30, 40)** and the component **(16, 17, 33).** During electrical discharge from the discharge part **(30, 40),** the insulating medium **(60)** obstructs electrical discharge generated from the discharge part **(30, 40)** towards the component **(16, 17, 33).** Accordingly, even where the distance between the component **(16, 17, 33)** and the discharge part **(30, 40)** is made narrow, it is possible for the discharge part **(30, 40)** to generate a desired electrical discharge.

The present invention provides, as a second aspect according to the first aspect, an air purification device which is characterized in that the discharge part **(30, 40)** is formed by an ionization part **(30)** for electrically charging dust particles present in the air.

In the second aspect of the present invention, corona discharge is generated in the ionization part **(30)** as a discharge part, whereby airborne dust particles are electrically charged. The dust particles thus electrically charged are trapped by a dust collecting electrode or by an electrostatic filter, whereby the airborne dust particles are removed. During corona discharge from the ionization part **(30),** the insulating medium **(60)** obstructs electrical discharge generated from the ionization part **(30)** towards the component **(16, 17, 33).** Accordingly, even where the distance between the component **(16, 17, 33)** and the ionization part **(30)** is made narrow, it is possible for the ionization part **(30)** to generate a desired corona discharge.

The present invention provides, as a third aspect according to the second aspect, an air purification device which is characterized in that the ionization part **(30)** comprising a discharge electrode **(31)** which serves as a discharge base end and a counter electrode **(32)** which serves as a discharge terminal end is configured such that the discharge and counter electrodes **(31, 32)** are arrayed in a direction perpendicular to the flow of the air, and that the insulating medium **(60)** is disposed between the component **(16, 17, 33)** adjacent to the ionization part **(30)** on either the upstream side or the downstream side thereof and the discharge electrode **(31).**

In the third aspect of the present invention, corona discharge is generated between the discharge electrode **(31)** and the counter electrode **(32).** The direction in which the corona discharge is generated is perpendicular to the direction of air flow. This therefore improves the efficiency of contact between ion wind caused by electrical discharge and air, thereby making it possible to improve the capability of collecting dust particles. In addition, the insulating medium **(60)** obstructs electrical discharge from the discharge electrode **(31)** to the component **(16, 17, 33)** on either the upstream side or the downstream side. Accordingly, even where the distance between the component **(16, 17, 33)** and the ionization part **(30)** is made narrow, corona discharge in a direction perpendicular to the flow of air is maintained in the ionization part **(30).**

The present invention provides, as a fourth aspect according to the third aspect, an air purification device which is characterized in that a dust collecting means **(17)** for trapping dust particles electrically charged by the ionization part **(30)** is disposed downstream of the ionization part **(30),** and that the insulating medium **(60)** is disposed between the dust collecting means **(17)** as the aforesaid component and the discharge electrode **(31).** Here, by "dust collecting means" is meant, for example, a dust collecting plate, a dust collecting electrode, or an electrostatic filter for electrically attracting electrically charged dust particles to trap them.

In the fourth aspect of the present invention, dust particles electrically charged by the ionization part **(30)** are made to flow on the downstream side of the ionization part **(30),** at which time the dust particles are trapped by the dust collecting means **(17).** The insulating medium **(60)** obstructs electrical discharge in the direction of air flow from the discharge electrode **(31)** to the dust collecting means **(17).** Therefore, even where the distance between the dust collecting means **(17)** and the ionization part **(30)** is made narrow, corona discharge in a direction perpendicular to the flow of air is maintained in the ionization part **(30).**

The present invention provides, as a fifth aspect according to the third aspect, an air purification device which is characterized in that a prefilter **(16)** for trapping dust particles present in the air is disposed upstream of the ionization part **(30),** and that the insulating medium **(60)** is disposed between the prefilter **(16)** as the aforesaid component and the discharge electrode **(31).**

In the fifth aspect of the present invention, on the upstream side of the ionization part **(30)**, dust particles of relatively large size present in the air to be treated are trapped by the prefilter **(16).** The insulating medium **(60)** obstructs electrical discharge in an opposite direction to the flow of air from the discharge electrode **(31)** to the prefilter **(16).** Therefore, even where the distance between the prefilter **(16)** and the ionization part **(30)** is made narrow, corona discharge in a direction perpendicular to the flow of air is maintained in the ionization part **(30).**

The present invention provides, as a sixth aspect according to the third aspect, an air purification device which is characterized in that on the downstream side of the ionization part **(30)** an electrostatic filter **(17)** for trapping dust particles electrically charged by the ionization part **(30)** and an earth electrode **(33)** for providing isolation between the discharge electrode **(31)** and the electrostatic filter **(17)** are disposed, and that the insulating medium **(60)** is disposed between the earth electrode **(33)** as the aforesaid component and the discharge electrode **(31).**

In the sixth aspect of the present invention, dust particles electrically charged by the ionization part **(30)** are made to flow on the downstream side of the ionization part **(30),** at which time the dust particles are attracted towards the electrostatic filter **(17)** by coulomb force and trapped there. The ionization part **(30)** and the electrostatic filter **(17)** are isolated from each other by the earth electrode **(33).** Consequently, for example, even when electrical discharge is generated in a direction from the ionization part **(30)** to the electrostatic filter **(17),** the electrical discharge flows through the earth electrode **(33)** towards the earth side. In other words, the earth electrode **(33)** inhibits electrical discharge such a spark from the ionization part **(30)** to the electrostatic filter **(17)** without fail, thereby protecting the electrostatic filter **(17).**

In the case where the earth electrode **(33)** is disposed between the ionization part **(30)** and the electrostatic filter **(17)** as described above, the ionization part **(30)** and the earth electrode **(33)** lie adjacent to each other. As a result, electrical discharge from the discharge electrode **(31)** concentrates in the earth electrode **(33)** while on the other hand there takes place little electrical discharge from the discharge electrode **(31)** to the counter electrode **(32),** thereby giving rise to the possibility that dust particles may not be effectively electrically charged. To cope with this, in the present aspect, the insulating medium **(60)** is disposed between the ionization part **(30)** and the earth electrode **(33).** The insulating medium **(60)** obstructs electrical discharge in the direction of air flow from the discharge electrode **(31)** to the earth electrode **(33).** Accordingly, even where the distance between the earth electrode **(33)** and the ionization part **(30)** is made narrow, corona discharge in a direction perpendicular to the flow of air is generated in the ionization part **(30).**

The present invention provides, as a seventh aspect according to the sixth aspect, an air purification device which is characterized in that the insulating medium **(60)** is so disposed as to cover a part of an upstream-side surface of the earth electrode **(33).**

In the seventh aspect of the present invention, the earth electrode **(33)** is partially (relative to the discharge electrode **(31))** covered by the insulating medium **(60)** while on the other hand the rest of the earth electrode **(33)** is exposed to the discharge electrode **(31).**

Incidentally, if the earth electrode **(33)** is completely (relative to the discharge electrode **(31))** covered by the insulating medium **(60),** substantially every electrical discharge from the discharge electrode **(31)** is directed to the counter electrode **(32).** In this case, however, the insulating medium **(60)** adjacent to the discharge electrode **(31)** becomes electrically charged, thereby increasing the density of electrical line force of the corona discharge from the discharge electrode **(31)** to the counter electrode **(32).** As a result, the voltage of electrical discharge of the discharge electrode **(31)** tends to excessively increase. Such a rise in the voltage of electrical discharge is undesirable from a point of view of the insulating design of the air purification device.

On the other hand, as in the present aspect, a certain part of the earth electrode **(33)** is exposed to the discharge electrode **(31),** whereby some degree of electrical discharge from the discharge electrode **(31)** to the earth electrode **(33)** is allowed. Accordingly, the aforesaid rise in the density of electrical line force is suppressed and consequently the rise in the voltage of electrical discharge is suppressed. The insulating medium **(60)** obstructs electrical discharge between the discharge electrode **(31)** and the part of the earth electrode **(33),** whereby even when the distance between the earth electrode **(33)** and the ionization part **(30)** is made narrow, corona discharge in a direction perpendicular to the flow of air is maintained in the ionization part **(30).**

The present invention provides, as an eighth aspect according to either the sixth aspect or the seventh aspect, an air purification device which is characterized in that the discharge electrode **(31)** is formed into a linear shape or into a rod-like shape, that the counter electrode **(32)** is shaped like a plate facing an outer peripheral surface of the discharge electrode **(31)** and is disposed on each side of the discharge electrode **(31),** that the earth electrode **(33)** is shaped like a plate having a plurality of openings and is in continuous form with downstream-side ends of the counter electrodes **(32),** and that the insulating medium **(60)** is supported on an upstream-side surface of the earth electrode **(33).**

In the eighth aspect of the present invention, the discharge electrode **(31)** formed in a linear or rod-like shape is interposed between the pair of counter electrodes **(32),** while each of the counter electrodes **(32)** and the discharge electrode **(31)** are arrayed in a direction perpendicular to the flow of air. Accordingly, in the ionization part **(30),** corona discharge is generated from the outer peripheral surface of the discharge electrode **(31)** to the flat surface of the counter electrode **(32).**

In addition, the earth electrode **(33)** is placed between the downstream-side ends of the counter electrodes **(32)** and is in continuous form with the counter electrodes (32). In other words, the counter electrodes **(32)** and the earth electrode **(33)** are formed integrally with each other. The earth electrode **(33)** inhibits electrical discharge from the discharge electrode **(31)** to the electrostatic filter **(17).**

Furthermore, the insulating medium **(60)** is supported on the upstream-side surface of the earth electrode **(33)** and obstructs electrical discharge from the discharge electrode **(31)** to the earth electrode **(33).** Accordingly, even where the distance between the earth electrode **(33)** and the ionization part **(30)** is made narrow, corona discharge in a direction perpendicular to the flow of air is maintained in the ionization part **(30).**

The present invention provides, as a ninth aspect according to any one of the first to eighth aspects, an air purification device which is characterized in that the aforesaid insulating medium is composed of a resinous material **(60)** having insulating properties.

In the ninth aspect of the present invention, the insulative resinous material **(60)** obstructs electrical discharge from the discharge part **(30, 40)** such as an ionization part et cetera to the component such as an electrostatic filter, earth electrode et cetera.

### ADVANTAGEOUS EFFECTS OF THE INVENTION

In accordance with the present invention, the insulating medium **(60)** is disposed between the discharge part **(30, 40)** and the other component **(16, 17, 33)** adjacent to the discharge part **(30, 40),** whereby even when the component **(16, 17, 33)** and the discharge part **(30, 40)** are in close proximity with each other electrical discharge from the discharge part **(30, 40)** to the component **(16, 17, 33)** is obstructed. This therefore makes it possible to maintain the discharge capability of the discharge part **(30, 40)** while accomplishing downsizing and low-profiling of the air purification device by reducing the distance between the discharge part **(30, 40)** and the other component **(16, 17, 33).**

In accordance with the second aspect of the present invention, especially with respect to the ionization part **(30)** for electrical dust collection, electrical discharge from the ionization part **(30)** to the component **(16, 17, 33)** is obstructed by the insulating medium **(60).** This therefore makes it possible to generate a desired corona discharge in the ionization part **(30)** while accomplishing downsizing and low-profiling of the air purification device by reducing the distance between the discharge part **(30, 40)** and the other component **(16, 17, 33).**

In accordance with the third aspect of the present invention, in the ionization part **(30),** corona discharge is generated in a direction perpendicular to the flow of air to be treated. This therefore makes it possible to effectively electrically charge airborne dust particles, thereby improving the dust collecting efficiency of the air purification device. The insulating medium **(60)** is disposed such that electrical discharge from the discharge electrode **(31)** to the component **(16, 17, 33)** disposed in the direction of air flow or in the opposite direction to the flow of air is obstructed. This therefore makes it possible to accomplish low-profiling of the air purification device in the direction of air flow and, in addition, to maintain corona discharge in a direction perpendicular to the flow of air in the ionization part **(30).**

In accordance with the fourth aspect of the present invention, dust particles electrically charged in the ionization part **(30)** are trapped by the dust collecting means **(17),** thereby making it possible to effectively obtain dust collecting efficiency for the air purification device. Since the insulating medium **(60)** obstructs electrical discharge from the ionization part **(30)** to the dust collecting means **(17),** this makes it possible to accomplish low-profiling of the air purification device in the direction of air flow while maintaining corona discharge in the ionization part **(30).**

In accordance with the fifth aspect of the present invention, airborne dust particles of relatively large size are trapped by the prefilter **(16).** Thereafter, dust particles of relatively small size still remaining in the air are electrically charged and trapped in the ionization part **(30).** This therefore prevents dust particles of relatively large size from adhering to the discharge and counter electrodes **(31, 32),** and the dust collecting efficiency of the ionization part **(30)** is inhibited from falling. Since the insulating medium **(60)** obstructs electrical discharge from the ionization part **(30)** to the prefilter **(16),** this makes it possible to accomplish low-profiling of the air purification device in the direction of air flow while maintaining corona discharge in the ionization part **(30).**

In accordance with the sixth aspect of the present invention, dust particles electrically charged by the ionization part **(30)** are trapped by the electrostatic filter **(17),** thereby making it possible to further effectively obtain dust collecting efficiency for the air purification device. The earth electrode **(33)** is disposed between the discharge electrode **(31)** and the electrostatic filter **(17),** thereby making it possible to prevent electrical discharge (e.g., spark) from the discharge electrode **(31)** to the electrostatic filter **(17)** without fail. This ensures that the electrostatic filter **(17)** is protected, and that the reliability of the air purification device is enhanced.

If the earth electrode **(33)** is disposed in the vicinity of the discharge electrode **(31),** electrical discharge from the discharge electrode **(31)** to the earth electrode **(33)** tends to take place. In accordance with the present aspect, however, the insulating medium **(60)** obstructs electrical discharge from the discharge electrode **(31)** to the earth electrode **(33),** thereby making it possible to accomplish low-profiling of the air purification device in the direction of air flow while maintaining corona discharge in the ionization part **(30).**

In accordance with the seventh aspect of the present invention, the insulating medium **(60)** is disposed such that the earth electrode **(33)** is covered partially relative to the discharge electrode **(31),** whereby electrical discharge from the discharge electrode **(31)** to the exposed portion of the earth electrode **(33)** is allowed simultaneously with corona discharge from the discharge electrode **(31)** to the counter electrode **(32).** Although, if the earth electrode **(33)** is completely (relative to the discharge electrode **(31))** covered by the insulating medium **(60),** this causes an abrupt rise in the voltage of electrical discharge of the ionization part **(30).** Contrary to this, such a rise is suppressed in the present aspect. In other words, corona discharge from the discharge electrode **(31)** to the counter electrode **(32)** and electrical discharge from the discharge electrode **(31)** to the earth electrode **(33)** are balanced with each other, thereby making it possible to further effectively inhibit rise in the voltage of electrical discharge without diminishing the dust collecting capability of the ionization part **(30).** This therefore makes it possible to facilitate the insulating design of the air purification device.

In accordance with the eighth aspect of the present invention, the counter electrode **(32)** is disposed on each side of the discharge electrode **(31).** This therefore makes it possible to generate corona discharge from the discharge electrode **(31)** as a base point in both directions perpendicular to the direction of air flow, whereby airborne dust particles are effectively electrically charged.

In addition, the counter electrodes **(32)** are connected together, with the earth electrode **(33)** interposed therebetween, whereby the counter electrodes **(32)** and the earth electrode **(33)** are formed as an integral member. This therefore facilitates processing and handling of the counter and earth electrodes **(32, 33).** In addition, the earth electrode **(33)** is provided with a plurality of openings, whereby the loss of pressure associated with the flow of air is reduced and, in addition, airborne dust particles are effectively electrically charged by air disturbance.

Furthermore, the insulating medium **(60)** is supported on the upstream-side surface of the earth electrode **(33),** whereby mounting of the insulating medium **(60)** is facilitated and, in addition, the insulating medium **(60)** provides insulation between the discharge electrode **(31)** and the earth electrode **(33)** without fail.

In addition, in the case where corona discharge is generated from the outer periphery of the discharge electrode **(31)** formed in a linear shape to the counter electrode **(32),** generally electrical discharge tends to be generated over the entire circumference of the discharge electrode **(31).** Therefore, in the case where the earth electrode **(33)** is disposed in proximity to the linear discharge electrode **(31),** electrical discharge especially from the discharge electrode **(31)** to the earth electrode **(33)** tends to take place. On the other hand, in the present aspect, the insulating medium **(60)** obstructs electrical discharge from the linear discharge electrode **(31)** to the earth electrode **(33),** thereby making it possible to accomplish low-profiling of the air purification device without diminishing the electrical-discharge capability of the ionization part **(30).**

In accordance with the ninth aspect of the present invention, the insulating medium **(60)** is formed of resinous material having insulating properties, thereby facilitating processing and handling of the insulating medium **(60).** Besides, the insulating medium **(60)** can be inexpensively manufactured.

### BRIEF DESCRIPTION OF THE DRAWINGS

In the accompanying drawings:
Figure **1** is a schematic perspective view showing the overall arrangement of an air purification device according to an embodiment of the present invention;
Figure **2** is a schematic block diagram of the inside of the air purification device of the embodiment when viewed from above;
Figure **3** is a schematic block diagram of a streamer discharge part of the embodiment when viewed from above;
Figure **4** is a schematic perspective view of the streamer discharge part of the embodiment;
Figure **5** is a horizontal cross sectional view of an ionization part of the embodiment;
Figure **6** is a graph representing the comparison of the dust collecting capability of the ionization part of the embodiment with the dust collecting capability of a conventional ionization part;
Figure **7** is a graph representing the comparison in discharge voltage between when the earth electrode is partially covered by an insulating medium and when the earth electrode is completely covered by an insulating medium;
Figure **8** is a horizontal cross sectional view of an ionization part according to a variation of the embodiment; and
Figure **9** is a schematic perspective view showing the overall arrangement of a conventional air purification device.

### REFERENCE NUMERALS IN THE DRAWINGS

10: air purification device
15: air passageway
16: prefilter
17: electrostatic filter (dust collecting means)
30: ionization part (discharge part)
31: ionization wire (discharge electrode)
32: first electrode plate (counter electrode)
33: earth electrode
40: streamer discharge part (discharge part)
60: insulating medium

### BEST EMBODIMENT MODE FOR CARRYING OUT THE INVENTION

In the following, a preferred embodiment of the present invention will be described in detail with reference to the accompanying drawings.

An air purification device **(10)** according to the present embodiment is an air purification device for consumer applications (general household, small commercial stores et cetera). The air purification device **(10)** is intended to remove airborne matter present in air to be treated, such as dust particles, allergens, odorous components, harmful components et cetera, whereby room air is purified.

### ENTIRE CONFIGURATION OF AIR PURIFICATION DEVICE

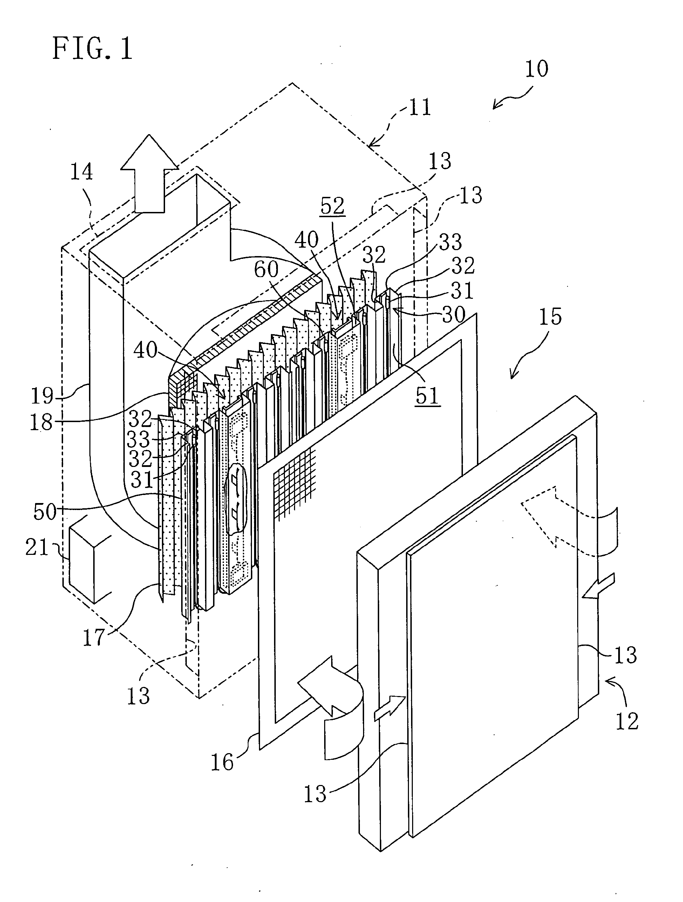

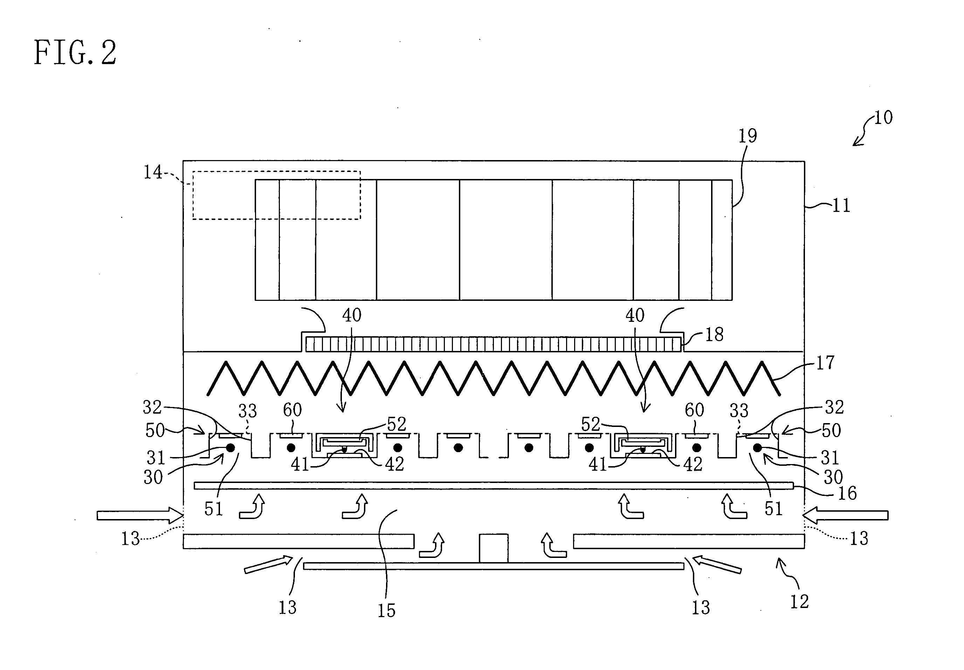

In the first place, description will be made in regard to the entire configuration of the air purification device **(10)** by making reference to Figures **1** and **2.** Figure **1** is an exploded perspective view of the air purification device **(10).** Figure **2** is a view of the inside of the air purification device **(10)** when viewed from above.

The air purification device **(10)** has a casing **(11)** one end surface of which is opened and a front cover **(12)** mounted over the opened end surface of the casing **(11).** Air inlet openings **(13)** through which room air is introduced into the casing **(11)** are formed in the casing **(11).** More specifically, the air inlet openings **(13)** are provided respectively in positions of the right, left, and upper surfaces of the casing **(11)** situated on the side of the front cover **(12)** and in a central part of the front cover **(12).** In addition, an air outlet opening **(14)** through which the room air flows out is formed in a position of a top plate of the casing **(11)** situated on the rear side.

In the casing **(11),** an air passageway **(15)** through which room air (i.e., air to be treated) flows is so formed as to extend from the air inlet openings **(13)** to the air outlet opening **(14).** Disposed sequentially in the upstream to downstream direction of the flow of room air in the air passageway **(15)** are a prefilter **(16),** an ionization part **(30),** a streamer discharge part **(40),** an electrostatic filter **(17),** a catalytic filter **(18),** and a centrifugal air blower **(19).** In addition, a power supply means **(21)** for both the ionization part **(30)** and the streamer discharge part **(40)** is disposed on the side of the lower rear of the casing **(11).**

### CONFIGURATION OF PREFILTER AND IONIZATION PART

The prefilter **(16)** is a filter for trapping dust particles of relatively large size which are present in room air. The ionization part **(30)** is a part for electrically charging dust particles of relatively small size which are small enough to pass through the prefilter **(16).** The ionization part **(30)** is mounted on the front side of a corrugated member **(50)** formed in an horizontally extending shape having a series of horizontal cross sections of squared U-shape. In addition, in the present embodiment, two corrugated members **(50)** are arrayed horizontally in the air passageway **(15).**

Defined on the front side of the corrugated member **(50)** are a plurality of approximately columnar spaces divided by the corrugated member **(50)** and each of the columnar spaces constitutes a front-side open part **(51).** And each front-side open part **(51)** is provided with an ionization wire **(31)** and a first electrode plate **(32).** As shown in Figure 2, each ionization wire **(31)** and each first electrode plate **(32)** are arrayed in a direction perpendicular to the flow of air in such a relation that two first electrode plates **(32)** on the right and left sides associate with a single ionization wire **(31).**

The ionization wire **(31)** is disposed approximately centrally (relative to the horizontal direction) in the front-side open part **(51).** The ionization wire **(31)** is formed in a linear or rod-like shape, and extends from the upper to the lower end of the corrugated member **(50).** The ionization wire **(31)** constitutes a discharge electrode which becomes a corona discharge's base end for electrically charging dust particles.

The first electrode plate **(32)** is formed by right and left side walls of the front-side open part **(51)** and is disposed on each side of its associated ionization wire **(31).** Each first electrode plate **(32)** is shaped like a plate facing the outer peripheral surface of the ionization wire **(31)** and is oriented in approximately parallel with the ionization wire **(31).** The first electrode plate **(32)** constitutes a counter electrode which becomes a corona discharge's terminal end.

Each of the most downstream-side surfaces of the corrugated member **(50)** constitutes an earth electrode **(33)** which becomes a component adjacent to the ionization wire **(31).** The earth electrode **(33)** is located between the ionization wire **(31)** and the electrostatic filter **(17)** so that it provides isolation between the ionization wire **(31)** and the electrostatic filter **(17).** And the earth electrode **(33)** inhibits electrical discharge such as a spark from the ionization wire **(31)** to the electrostatic filter **(17),** thereby protecting the electrostatic filter **(17).** In addition, each earth electrode **(33)** is formed in a mesh, net-like, or punching metal shape. In other words, the earth electrode **(33)** is shaped like a plate having a plurality of openings. The earth electrode **(33)** is in continuous form with the downstream ends of the right and left first electrode plates **(32)** corresponding to the ionization wire **(31).** And the first electrode plates **(32)** and the earth electrodes **(33)** together constitute the integrated corrugated member **(50).** In addition, an insulating medium **(60)** is supported on the upstream surface of the earth electrode plate **(33). CONFIGURATION OF STREAMER DISCHARGE PART**

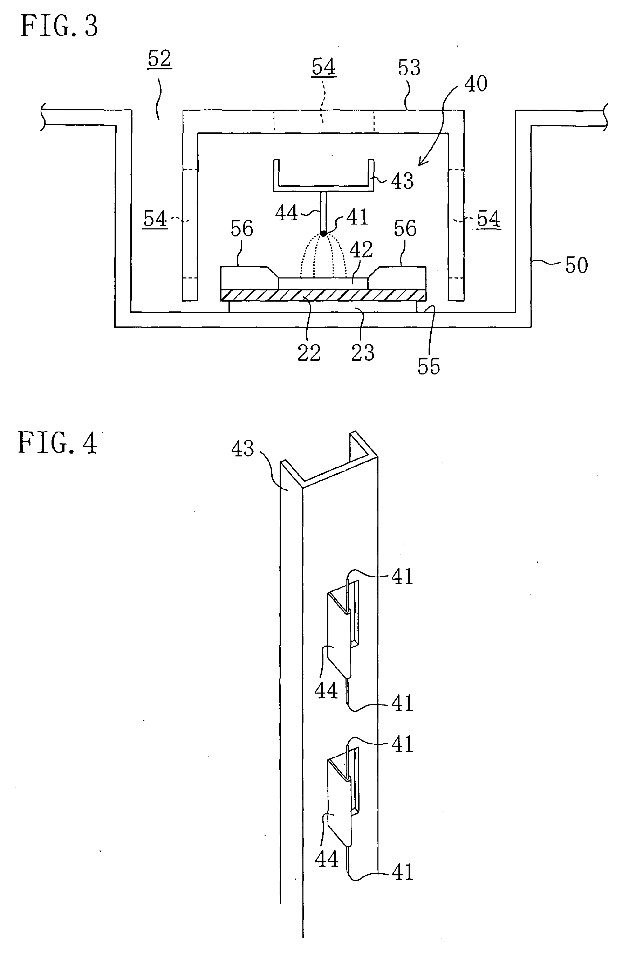

The streamer discharge part **(40)** is mounted on the rear side of the corrugated member **(50).** More specifically, defined on the rear side of the corrugated member **(50)** are a plurality of approximately columnar spaces divided by the corrugated member **(50).** Of these columnar spaces, two columnar spaces of larger horizontal cross sectional area each constitute a rear-side open part **(52).** The streamer discharge part **(40)** is provided in each of these two rear-side open parts **(52).** In addition, as shown in Figure 3 depicting a chief section of the streamer discharge part **(40)** when viewed from above, the rear-side open part **(52)** is provided with a first insulating cover **(53)** having a horizontal cross section of squared U-shape and extending vertically across the corrugated member **(50).** Defined on the front side of the first insulating cover **(53)** is an open space. And the streamer discharge part **(40)** is encapsulated between the rear surface of the corrugated member **(50)** and the inside surface of the first insulating cover **(53).** In addition, three wall surfaces together constituting the first insulating cover **(53)** are each provided with a plurality of air circulation openings, whereby room air is allowed to circulate in the vicinity of the streamer discharge part **(40).**

The streamer discharge part **(40)** is provided with a plurality of electrode pairs **(41, 42).** Each of the electrode pairs **(41, 42)** is made up of a plurality of discharge needles **(41)** serving as streamer discharge's base ends and a plurality of second electrode plates **(42)** serving as streamer discharge's terminal ends.

As shown in Figure **4** (which is a perspective view illustrating in an enlarged manner a chief section of the discharge needle), the discharge needles **(41)** are supported on an electrode holding member **(43)** having a horizontal cross section of squared U-shape and extending vertically. More specifically, a plurality of supporting plates **(44)** which are curvature-formed forwardly are provided in predetermined positions of the electrode holding member **(43).** And the discharge needle **(41)** formed in a linear or rod-like shape is supported by the front edge of the supporting plate **(44)** caulked to pinch the discharge needle **(41)** therein. As a result of such arrangement, both of the ends of the discharge needle **(41)** project out vertically from the supporting plate **(44).** In the present embodiment, the discharge needle **(41)** is formed of a tungsten wire having a wire diameter of about 0.2 millimeter.

As shown in Figure **3**, the second electrode plate **(42)** is formed in a supporting surface **(55)** which is one of internal walls defining the rear-side open part **(52)** of the corrugated member **(50)** that is located in front of the discharge needle **(41).** More specifically, the second electrode plate **(42),** a high resistance resinous sheet **(22),** and a conducting plate **(23)** are layered one upon the other in order of nearness to the discharge needle **(41).** The second electrode plate **(42)** is shaped like a vertically extending plate. The second electrode plate **(42)** is provided, in its predetermined position, with a second insulating cover **(56).** And the second electrode plate **(42)** is exposed at a surface thereof facing the tip of the discharge needle **(41).**

In the way as described above, the discharge needle **(41)** and the second electrode plate **(42)** are oriented such that they are substantially in parallel with each other. And the distance between the tip of the discharge needle **(41)** and the second electrode plate **(42)** is maintained at a predetermined interval. In addition, in the present embodiment, the distance between the electrodes **(41, 42)** is 4.0±0.3 millimeters.

### CONFIGURATION OF ELECTROSTATIC FILTER AND CATALYTIC FILTER

The electrostatic filter **(17)** constitutes a dust collecting means of the present invention which is disposed downstream of the ionization part **(30)** and the streamer discharge part **(40).** The electrostatic filter **(17)** has an upstream-side surface and a downstream-side surface the former of which constitutes a dust collecting surface for trapping dust particles of relatively small size which are electrically charged by the ionization part **(30)** and the latter of which supports thereon a photocatalyst (optical semiconductor). The photocatalyst is further activated by substances of high reactivity (e.g., active species such as electron, ion, radical hydroxide) present in a low temperature plasma generated by streamer discharge by the streamer discharge part **(40),** thereby promoting decomposition of harmful components and odorous components present in room air. In addition, as the photocatalyst, titanium dioxide, zinc oxide, tungsten oxide, or cadmium sulfide may be used. In addition, the electrostatic filter **(17)** is formed by a so-called "pleated filter" having a wavily bending horizontal cross section.

The catalytic filter **(18)** is disposed downstream of the electrostatic filter **(17).** In the catalytic filter **(18),** a plasma catalyst is supported on the surface of a substrate of honeycomb structure. The plasma catalyst is further activated by substances of high reactivity (e.g., active species such as electron, ion, radical hydroxide) present in a low temperature plasma generated by electrical discharge by the streamer discharge part **(40),** thereby promoting decomposition of harmful components and odorous components present in room air. As the plasma catalyst, catalysts of the manganese family, catalysts of the precious metal family, or catalysts prepared by addition of an adsorbent to the aforesaid catalysts.

### DETAILED STRUCTURE OF INSULATING MEDIUM AND IONIZATION PART

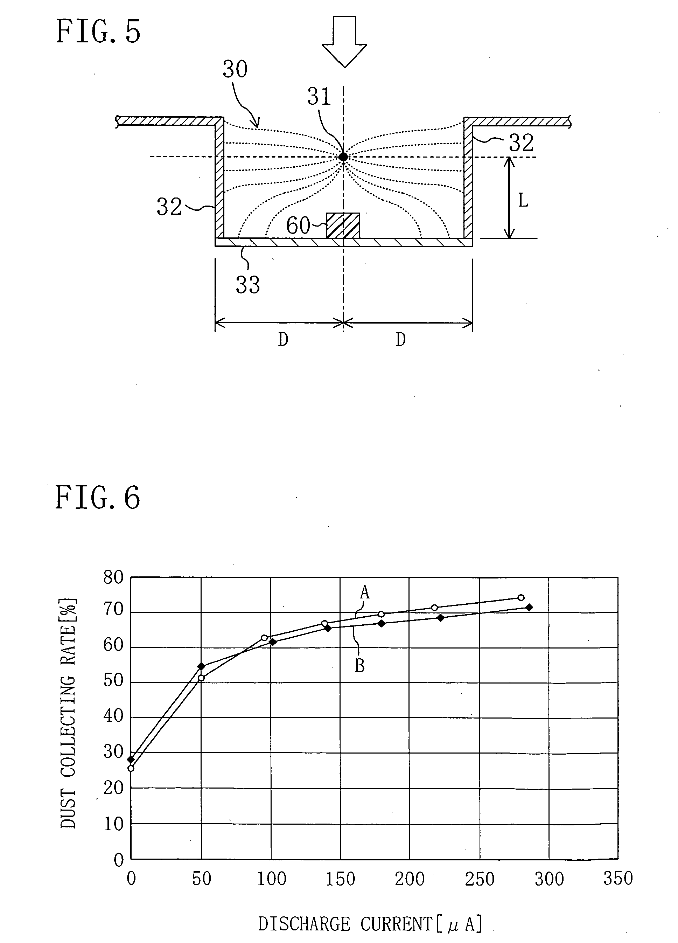

Referring now to Figure 5, the insulating medium **(60)** and the ionization part **(30)** are described in regard to their respective detailed structures. As described above, the insulating medium **(60)** is supported on the upstream-side surface of the earth electrode **(60).** The insulating medium **(60)** is formed of a resinous material having insulating properties. In addition, the insulating medium **(60)** is shaped like a plate or rectangular parallelepiped extending from the upper to the lower end of the corrugated member **(50).**

More specifically, the thickness dimension of the insulating medium **(60)** (vertical dimension in Figure **5)** is about 3 millimeters and the width dimension (horizontal dimension in Figure **5)** is about 6 millimeters. And the insulating medium **(60)** is disposed such that its widthwise center is located substantially centrally between a pair of first electrode plates **(32, 32).** In addition, the distance between the first electrode plates **(32, 32)** is about 24 millimeters. Accordingly, with respect to the ionization wire **(31),** an about 6-mm central portion of the earth electrode **(33)** is covered by the insulating medium **(60).** On the other hand, about 9-mm portions of the earth electrode **(33)** situated on both sides of the insulating medium **(60)** are exposed to the ionization wire **(31).**

In addition, in the present embodiment, the distance (D) from the ionization wire **(31)** to the first electrode plate **(32)** is about 12 millimeters and the distance (L) from the ionization wire **(31)** to the earth electrode **(33)** is about 10 millimeters. Stated another way, the ionization part **(30)** is designed such that the distance (L) from the ionization wire **(31)** to the earth electrode **(33)** is shorter than the distance (D) between the ionization wire **(31)** and the first electrode plate **(32).**

### RUNNING OPERATION

As shown in Figures **1** and **2,** during operation of the air purification device **(10),** the centrifugal air blower **(19)** is operated, whereby room air flows through the air passageway **(15)** within the casing **(11).** In this state, voltages are applied to the ionization part **(30)** as well as to the streamer discharge part **(40)** from the power supply means **(21).**

Room air introduced into the casing **(11)** first passes through the prefilter **(16).** In the prefilter **(16),** dust particles of relatively large size which are present in the room air are removed. Thereafter, the room air flows to the ionization part **(30)** and then to the streamer discharge part **(40).**

In the ionization part **(30),** corona discharge is generated to the first electrode plates **(32)** on the both sides of the ionization wire **(31)** from the ionization wire **(31).** In other words, in the ionization part **(30),** electrical discharge is generated in a direction perpendicular to the flow of air. As a result, dust particles of relatively small size which are present in the room air are electrically charged. The dust particles thus electrically charged are trapped at the upstream-side dust collecting surface of the electrostatic filter **(17)** during passage therethrough.

In the streamer discharge part **(40),** a low temperature plasma is generated by streamer discharge generated between the discharge needle **(41)** and the second electrode plate **(42).** This low temperature plasma contains substances of high reactivity (active species) such as ozone. The active species contact with room air and decompose harmful components and odorous components present in the room air.

Thereafter, the room air passes through the electrostatic filter **(17).** As described above, dust particles are trapped at the dust collecting surface of the electrostatic filter **(17)** and the active species are further activated by the photocatalyst supported on the downstream-side surface thereof. Consequently, harmful components and odorous components present in the room air are decomposed to a further extent.

Thereafter, the room air passes through the catalytic filter **(18).** In the catalytic filter **(18),** the active species are further activated, whereby harmful components and odorous components present in the room air are decomposed to a still further extent. In addition, in the catalytic filter **(18),** odorous components and harmful components remaining in the room air are subjected to an adsorptive treatment. The room air now purified in the way as described above is drawn into the centrifugal air blower **(19)** and then discharged into the room through the air outlet opening **(14).**

### VERIFICATION OF ELECTRICAL DISCHARGE OF IONIZATION PART

Incidentally, in the ionization part **(30)** of the present embodiment, the distance (L) between the ionization wire **(31)** and the earth electrode **(33)** is designed to be shorter than the distance (D) between the ionization wire **(31)** and the first electrode plate **(32)** (see Figure **5),** thereby accomplishing low-profiling of the air purification device. However, if the distance (L) between the ionization wire **(31)** and the earth electrode **(33)** is made narrow as described above (especially if L < D), the amount of electrical discharge from the ionization wire **(31)** to the earth electrode **(33)** increases while on the other hand the amount of electrical discharge from the ionization wire **(31)** to the first electrode plate **(32)** decreases, thereby resulting in reducing also the effect of electrically charging dust particles by the ionization part **(30).**

In the present embodiment, the insulating medium **(60)** is disposed between the ionization wire **(31)** and the earth electrode **(33),** whereby electrical discharge in the direction of air flow from the ionization wire **(31)** to the earth electrode **(33)** is obstructed by the insulating medium **(60).** As a result, in the ionization part **(30),** corona discharge from the ionization wire **(31)** to the first electrode plate **(32)** is maintained.

The above was experimentally verified and the results of such verification will be discussed with reference to a graph shown in Figure **6.** The graph of Figure **6** comparatively verifies the effect of electrically charging dust particles (the capability of collecting dust particles) by the ionization part **(30)** of the present embodiment and that by a conventional ionization part. In Figure **6,** the relationship between the dust collecting rate of the ionization part **(30)** of the present embodiment and the discharge current is represented by line A while on the other hand the relationship between the dust collecting rate of the conventional ionization part and the discharge current is represented by line B. Note that in the conventional ionization part the distance (D) from the ionization wire to the first electrode plate is about 12 millimeters as in the present embodiment while on the other hand the distance (L) from the ionization wire to the earth electrode is about 12 millimeters (longer than the present embodiment in which the distance (L) is about 8 millimeters). In addition, the conventional ionization part is not provided with an insulating medium of the type as provided in the present embodiment, in other words the earth electrode is disposed such that it is completely exposed to the ionization wire.

As can be seen from Figure **6,** although in the ionization part **(30)** of the present embodiment the distance (L) between the ionization wire **(31)** and the earth electrode **(33)** is about two thirds of the distance (L) in the conventional ionization part, almost the same dust collecting rate as the conventional ionization part or more than that is obtained. That is to say, in the present embodiment, it is possible to accomplish low-profiling of the ionization part **(30)** while at the same time maintaining the dust collecting capability of the ionization part **(30).**

In addition, it is arranged in the present embodiment such that a central part of the earth electrode **(33)** is covered by the insulating medium **(60).** As a result of such arrangement, other than electrical discharge from the ionization wire **(31)** to the first electrode plate **(32)** is generated, in other words electrical discharge from the ionization wire **(31)** to the earth electrode **(33)** exposed on each side of the insulating medium **(60)** is generated, as shown in Figure **5.** Consequently, for example, when compared to the case where the earth electrode **(33)** is completely covered by the insulating medium **(60),** the density of electrical line force of the ionization wire **(31)** can be further reduced, thereby making it possible to inhibit rise in the voltage of electrical discharge of the ionization part **(30).**

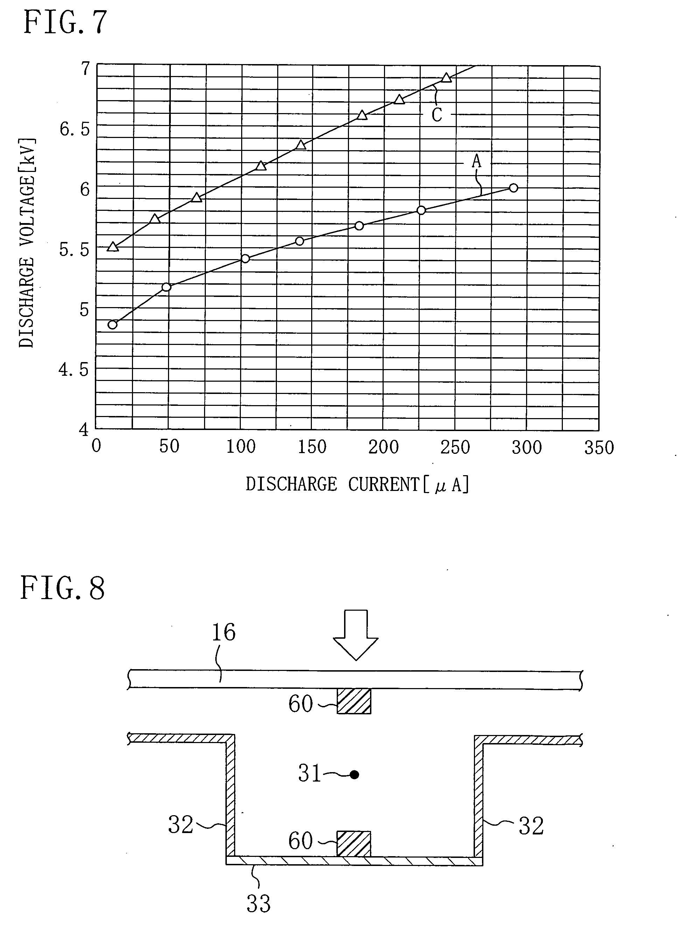

The above was experimentally verified and the results of such verification will be discussed with reference to a graph shown in Figure 7. The graph of Figure 7 comparatively verifies the relationship between discharge current and electrical-discharge voltage in regard to different ionization parts, i.e., the ionization part **(30)** (indicate by line A of Figure 7) of the present embodiment in which a central part of the earth electrode **(33)** is covered by the insulating medium **(60)** and the ionization part (indicated by line B of Figure 7) in which the earth electrode is entirely covered by the insulating medium.

As can be seen from Figure 7, when compared to the case where the entire earth electrode is covered by the insulating medium, the voltage of electrical discharge can be totally reduced in the ionization part **(30)** of the present embodiment. In other words, in the present embodiment, some degree of electrical discharge from the ionization wire **(31)** to the earth electrode **(33)** is allowed, thereby making it possible to inhibit rise in the density of electrical line force in the ionization part **(30),** as described above. Accordingly, as compared to the case where the earth electrode is completely covered by the insulating medium, it becomes possible to more effectively suppress rise in the voltage of electrical discharge.

### ADVANTAGEOUS EFFECTS OF THE EMBODIMENT

In accordance with the present embodiment, corona discharge is generated in a direction perpendicular to the flow of air to be treated in the ionization part **(30).** Consequently, it becomes possible to effectively electrically charge airborne dust particles, thereby making it possible to improve the dust collecting efficiency of the air purification device.

In addition, in accordance with the present embodiment, the earth electrode **(33)** is interposed between the ionization wire **(31)** and the electrostatic filter **(17).** This therefore ensures that electrical discharge from the ionization wire **(31)** to the electrostatic filter **(17)** is prevented without fail. Accordingly, the electrostatic filter **(17)** is protected without fail, thereby making it possible to enhance the reliability of the air purification device.

On the other hand, if the earth electrode **(33)** is disposed in the vicinity of the ionization wire **(31),** electrical discharge, especially from the ionization wire **(31)** to the earth electrode **(33),** is likely to occur. However, in accordance with the present embodiment, electrical discharge from the ionization wire **(31)** to the earth electrode **(33)** is obstructed by the insulating medium **(60).** This therefore makes it possible to accomplish low-profiling of the air purification device in the direction of air flow while maintaining corona discharge in a direction perpendicular to the flow of air in the ionization part **(30).** In addition, the insulating medium **(60)** is so disposed as to cover the central part of the earth electrode **(33),** whereby rise in the voltage of electrical discharge in the ionization part **(30)** is effectively suppressed. This therefore facilitates the insulating design of the air purification device.

In addition, in accordance with the present embodiment, the first electrode plate **(32)** and the earth electrode **(33)** are configured as the integrated corrugated member **(50).** This therefore facilitates processing and handling of the first electrode plate **(32)** and the earth electrode **(33).** Furthermore, the insulating medium **(60)** is supported on the upstream-side surface of the earth electrode **(33),** whereby the insulating medium **(60)** becomes easy to mount and, in addition, isolation between the ionization wire **(31)** and the earth electrode **(33)** is provided without fail. Additionally, the earth electrode **(33)** is provided with a plurality of openings, whereby pressure loss associated with the flow of air is reduced, and owing to turbulent air the efficiency of electrically charging duct particles by the ionization part **(30)** or the efficiency of contact between active species generated from the streamer discharge part **(40)** and odorous components et cetera present in air is enhanced.

### VARIATION OF THE EMBODIMENT

For example, in the air purification device of the aforesaid embodiment, the insulating medium **(60)** may be disposed between the ionization part **(30)** and the prefilter **(16)** positioned upstream of the ionization part **(30),** as shown in Figure **8.** In this arrangement, electrical discharge from the ionization wire **(31)** to the prefilter **(16)** is obstructed by the insulating medium **(60),** thereby making it possible to reduce the distance between the ionization wire **(31)** and the prefilter **(16)** while maintaining corona discharge in a direction perpendicular to the flow of air. In the example of Figure **8,** the insulating medium **(60)** is disposed also between the ionization wire **(31)** and the earth electrode **(33),** as in the aforesaid embodiment. Alternatively, it may be arranged such that the insulating medium **(60)** is disposed only between the ionization wire **(31)** and the prefilter **(16).**

In addition, for the case of no provision of the earth electrode **(33),** the insulating medium **(60)** may be disposed between the ionization part **(30)** and the electrostatic filter **(17)** serving as a component adjacent to the ionization part **(30).** For example, when employing the dust collecting means **(17)** (e.g., dust collecting plate, dust collecting electrode et cetera) as a substitute for an electrostatic filter, the insulating medium **(60)** may be disposed between the ionization part **(30)** and the dust collecting means **(17)** serving as a component adjacent to the ionization part **(30).**

### ANOTHER EMBODIMENT OF THE INVENTION

The aforesaid embodiment may be configured as follows.

In the aforesaid embodiment, the insulating medium **(60)** is disposed between the ionization part **(30)** for generating corona discharge and the component **(16, 17, 33)** adjacent to the ionization part **(30),** whereby electrical discharge from the ionization part **(30)** to the component **(16, 17, 33)** is obstructed. Alternatively, the insulating medium **(60)** may be disposed between the streamer discharge part **(40)** (not the ionization part **(30)**) and a component adjacent to the streamer discharge part **(40),** thereby obstructing electrical discharge from the streamer discharge part **(40)** to the component. Also in this arrangement, it is possible to accomplish low-profiling of the air purification device by reducing the distance between the streamer discharge part **(40)** and the other component while maintaining the electrical-discharge capability of the streamer discharge part **(40).** In addition, insulating mediums **(60)** of the same type as above may be applied to discharge parts which generate glow discharge or electrical discharges in other modes.

It should be noted that the above-descried embodiment is essentially preferable examples which are not intended in any sense to limit the scope of the present invention, its application, or its range of application.

### INDUSTRIAL APPLICABILITY

As has been described above, the present invention finds utility in the field of air purification devices having a discharge part for discharging electricity in air in which a stream of air to be treated flows.

## Claims

1. An air purification device which comprises:
an air passageway **(15)** through which air to be treated flows; and
a discharge part **(30, 40)** which discharges electricity in the air flowing through the air passageway **(15),**
wherein an insulating medium **(60)** is disposed between the discharge part **(30, 40)** and a component **(16, 17, 33)** adjacent to the discharge part **(30, 40).**

2. The air purification device of claim 1,
wherein the aforesaid discharge part is formed by an ionization part **(30)** for electrically charging dust particles present in the air.

3. The air purification device of claim 2,
wherein the ionization part **(30)** comprising a discharge electrode **(31)** which serves as a discharge base end and a counter electrode **(32)** which serves as a discharge terminal end is configured such that the discharge and counter electrodes **(31, 32)** are arrayed in a direction perpendicular to the flow of the air; and
wherein the insulating medium **(60)** is disposed between the component **(16, 17, 33)** adjacent to the ionization part **(30)** on either the upstream side or the downstream side thereof and the discharge electrode **(31).**

4. The air purification device of claim 3,
wherein dust collecting means **(17)** for trapping dust particles electrically charged by the ionization part **(30)** is disposed downstream of the ionization part **(30)**; and
wherein the insulating medium **(60)** is disposed between the dust collecting means **(17)** as the aforesaid component and the discharge electrode **(31).**

5. The air purification device of claim 3,
wherein a prefilter **(16)** for trapping dust particles present in the air is disposed upstream of the ionization part **(30);** and
wherein the insulating medium **(60)** is disposed between the prefilter **(16)** as the aforesaid component and the discharge electrode **(31).**

6. The air purification device of claim 3,
wherein on the downstream side of the ionization part **(30)** an electrostatic filter **(17)** for trapping dust particles electrically charged by the ionization part **(30)** and an earth electrode **(33)** for providing isolation between the discharge electrode **(31)** and the electrostatic filter **(17)** are disposed; and
wherein the insulating medium **(60)** is disposed between the earth electrode **(33)** as the aforesaid component and the discharge electrode **(31).**

7. The air purification device of claim 6,
wherein the insulating medium **(60)** is so disposed as to cover a part of an upstream-side surface of the earth electrode **(33).**

8. The air purification device of claim 6,
wherein the discharge electrode **(31)** is formed into a linear shape or into a rod-like shape;
wherein the counter electrode **(32)** is shaped like a plate facing an outer peripheral surface of the discharge electrode **(31)** and is disposed on each side of the discharge electrode **(31);**
wherein the earth electrode **(33)** is shaped like a plate having a plurality of openings and is in continuous form with downstream-side ends of the counter electrodes **(32);** and
wherein the insulating medium **(60)** is supported on an upstream-side surface of the earth electrode **(33).**

9. The air purification device of any one of claims 1-8,
wherein the aforesaid insulating medium is composed of a resinous material **(60)** having insulating properties.
